# EUROPEAN PATENT APPLICATION

(11) **EP 3 009 064 A1**
(43) Date of publication of application: **20.04.2016**
(21) Application number: 14810606.5
(22) Date of filing: 13.06.2014
(51) Int. Cl.: A61B 3/10

(54) **OPTICAL TOMOGRAPHY DEVICE**

(30) Priority: 14.06.2013 JP 2013125404
(71) Applicant: National University Corporation Nagoya University, Aichi 464-8601 (JP); Nidek Co., Ltd., Gamagori-shi, Aichi 443-0038 (JP)
(72) Inventor: TERASAKI, Hiroko, Nagoya-shi Aichi 464-8601 (JP); KOBAYASHI, Masahiko, Gamagori-shi Aichi 443-0038 (JP); NISHIYAMA, Junpei, Gamagori-shi Aichi 443-0038 (JP)
(74) Representative: Winter, Brandl, Fürniss, Hübner, Röss, Kaiser, Polte - Partnerschaft mbB
(86) International application number: PCT/JP2014/065768
(87) International publication number: WO 2014/200093

(57) **Abstract**

An optical tomography device that enables to confirm, by naked eyes, a position at which an optical tomographic image is to be captured is provided.

An optical tomography device is an optical tomography device that includes a first light source; and an optical device configured to guide first light from the first light source to a measurement target eye and detect interference light obtained from reflected light from the measurement target eye. The optical tomography device further includes: a second light source configured to emit aiming light indicative of a position to which the first light is applied; and an optical probe having an opening at a distal end and configured capable of being inserted in an eyeball. The optical probe applies the first light guided by the optical device, into the measurement target eye through the opening, guides the reflected light, which has been reflected by the measurement target eye, to the optical device, and applies, though the opening, the aiming light from the second light source to the position to which the first light is applied.

## Description

### Technical Field

### CROSS-REFERENCE TO RELATED APPLICATION

This application is based upon and claims the benefit of priority of Japanese Patent application No. 2013-125404, filed on June 14, 2013, the entire contents of which are incorporated herein by reference.

The technology disclosed herein relates to an optical tomography device which captures an optical tomographic image by use of optical coherence.

### Background Art

In recent years, devices that capture optical tomographic images of eyes by using optical coherence have been developed. For example, Patent Literature 1 discloses an image capturing device which obtains, by using separate optical systems, a fundus image and an optical tomographic image of an eye to be examined, and displays these two images associated with each other. In this image capturing device, first, the fundus image is displayed on a monitor, and a measurement line indicating a measurement position is additionally shown on the displayed fundus image. An examiner moves the measurement line on the fundus image while viewing the monitor, and sets a position at which to capture an optical tomographic image. Then, through operation by the examiner, an optical tomographic image at the position on the measurement line is captured.

### Citation List

Patent Literature 1: Japanese Patent Application Publication No. 2012-81330

### Summary of Invention

### Technical Problem

An image capturing device of this type is considered to be used in surgical treatment of an eye. That is, consideration of use is being given to providing necessary treatment while confirming a treatment site on an optical tomographic image during surgery of an eye. By using such an optical tomography device, a state (a state of retinal detachment, for example) that cannot be confirmed with naked eyes can be confirmed, and thus, appropriate treatment can be provided. In the case where the optical tomography device is used in surgical treatment, if light can be applied into an eyeball from an optical probe inserted in the eyeball, such would be preferable since an optical tomographic image at a desired position in the eyeball can be obtained. However, the light used in capturing the optical tomographic image is not visible light, and thus, it is necessary to confirm at which position in the eyeball the optical tomographic image is being captured. Due to this, employment of the technology of Patent Literature 1 in the optical tomography device may thus be considered. However, with the technology of Patent Literature 1, the examiner needs to operate the optical probe while viewing the fundus image displayed on the monitor, and to set the position at which to capture the optical tomographic image. In this case, it is difficult to grasp the relative distance between the optical probe and the image capturing site of the eye to be examined (retina, for example) based merely on the image displayed on the monitor. This may result in an increased possibility of the optical probe coming into contact with the image capturing site of the eye to be examined.

The present specification discloses an optical tomography device that can visually confirm, by naked eyes, the position at which an optical tomographic image is to be captured.

### Solution to Technical Problem

An optical tomography device disclosed in present specification is an optical tomography device comprising a first light source; and an optical device configured to guide first light from the first light source to a measurement target eye and detect interference light obtained from reflected light from the measurement target eye. The optical tomography device further comprises a second light source configured to emit aiming light indicative of a position to which the first light is applied; and an optical probe including an opening at a distal end and configured capable of being inserted in an eyeball. The optical probe applies the first light guided by the optical device, into the measurement target eye through the opening, guides the reflected light, which has been reflected by the measurement target eye, to the optical device, and applies, though the opening, the aiming light from the second light source to the position to which the first light is applied.

In the tomography device above, light (light for obtaining an optical tomographic image) from the first light source and the aiming light from the second light source are applied to the same position in the eyeball. Thus, an operator who operates the optical probe can apply the aiming light to a desired position by operating the optical probe while confirming, with one's naked eyes, the position to which the aiming light is applied, thereby capturing the optical tomographic image of the position. Therefore, the operator can confirm, with one's naked eyes, both the aiming light and the optical probe, and thus can easily grasp the relative distance between the optical probe and the capturing position, and can reduce the risk of unintended contact of the optical probe with the tissue in the eyeball.

The opening in the optical probe may be arranged on a side face of the optical probe in a predetermined angle range in a circumferential direction. The first light and the aiming light may be applied, through the opening, in a direction orthogonal to an axis of the optical probe, and may scan in the predetermined angle range about the axis of the optical probe. Moreover, the second light source may be configured capable of emitting the aiming light of a plurality of luminescent colors (red, green, etc., for example) and the optical tomography device may further be able to select, by a selecting switch, a luminescent color of the aiming light to be emitted from the second light source to the measurement target eye.

### Brief Description of Drawings

FIG. 1 shows a schematic configuration diagram of an optical system of an optical tomography device;
FIG. 2 shows a perspective view of an optical probe of the optical tomography device;
FIG 3 shows a vertical cross-sectional view of an eyeball with the optical probe of the optical tomography device inserted in the eyeball;
FIG 4 shows captured images of a normal retina of a domestic rabbit eye;
FIG 5 shows optical tomographic images of an optic nerve head of a domestic rabbit eye;
FIG 6 shows a state where a diamond eraser is in contact with the surface layer of a normal retina;
FIG 7 shows a state where a vitreous cortex and a part of a retina are aspirated and pulled by a back flush needle;
FIG 8 shows a state where a retina of a domestic rabbit eye has been detached;
FIG. 9 shows an optical tomographic image of pars plicate ciliaris of a pig eye;
FIG 10 shows optical tomographic images of a human epiretinal membrane with macular epiretinal membrane disease;
FIG. 11 shows a state where the epiretinal membrane is being treated in surgery;
FIG 12 shows optical tomographic images of vortex veins in choroid under human retina;
FIG 13 shows an optical tomographic image of ora serrata retinae which is the most peripheral part of human retina;
FIG. 14 shows optical tomographic images of human ciliary body;
FIG 15 shows an optical tomographic image of human intraocular lens, ciliary body, and iris; and
FIG. 16 shows an optical tomographic image of human cornea, iris, and iridocorneal angle.

### Description of Embodiments

### (Embodiment)

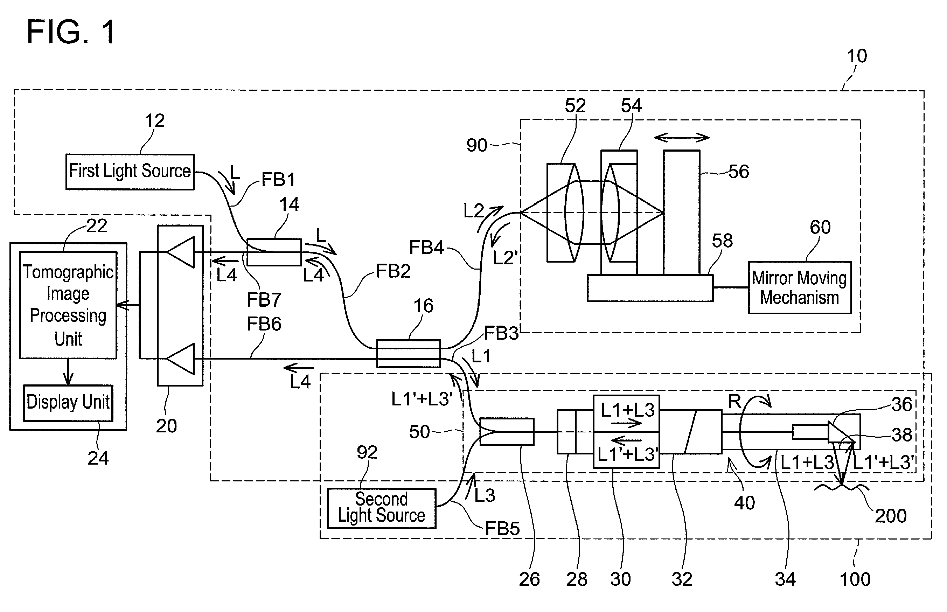

As shown in FIG. 1, an optical tomography device comprises: an interference optical system 10 which causes reflected light L1' from a measurement target eye 200 and reference light L2 to interfere with each other; and an irradiation optical system 100 which applies aiming light to the measurement target eye 200.

The interference optical system 10 comprises: a first light source 12; an optical probe 50 which applies measurement light L1 from the first light source 12 to the measurement target eye 200 and which receives the reflected light L1' from the measurement target eye 200; a reference optical system 90 which adjusts an optical path length of reference light L2 from the first light source 12; optical fibers FB1 to FB4, FB6, and FB7 which connect these components 12, 50, and 90; and optical fiber couplers 14 and 16.

The first light source 12 is a light source which emits light in a near-infrared range having high organism transmissivity. The first light source 12 is connected to the optical probe 50 and the reference optical system 90 via the optical fiber FB1, the optical fiber coupler 14, and the optical fibers FB2, FB3, and FB4. First light outputted from the first light source 12 is low coherence light and a wavelength thereof does not change over time. The first light L passes through the optical fiber FB1, the optical fiber coupler 14, and the optical fibers FB2, FB3, and FB4, to be guided to a measurement optical system and the reference optical system 90.

That is, the optical fibers FB1 to FB4, FB6, and FB7 are transmission lines which transmit light, and the optical fiber couplers 14 and 16 are devices which demultiplex and/or multiplex optical signals. One end of the optical fiber FB1 is connected to the first light source 12, whereas the other end thereof is connected to the optical fiber coupler 14. The optical fiber coupler 14 is connected to the optical fibers FB2 and FB7. The optical fiber coupler 14 outputs light inputted from the optical fiber FB1, to the optical fiber FB2, and outputs light inputted from the optical fiber FB2, to the optical fiber FB7. The optical fiber FB7 is connected to an interference light detector 20.

The optical fiber FB2 is connected to the optical fibers FB3 and FB4, and FB6 via the optical fiber coupler 16. The optical fiber coupler 16 demultiplexes light from the optical fiber FB2 to be outputted to the optical fibers FB3 and FB4. Also, the optical fiber coupler 16 multiplexes light inputted from the optical fibers FB3 and FB4, and demultiplexes the multiplexed light to be outputted to the optical fibers FB2 and FB6. The optical fiber FB3 is connected to the optical probe 50, the optical fiber FB4 is connected to the reference optical system 90, and the optical fiber FB6 is connected to the interference light detector 20.

The reference optical system 90 includes a first optical lens 52, a second optical lens 54, a reflective mirror 56, a base 58, and a mirror moving mechanism 60. The second optical lens 54 and the reflective mirror 56 are fixed to the base 58. The base 58 is provided so as to be able to reciprocate in the optical axis direction relative to the first optical lens 52. That is, the base 58 is connected to the mirror moving mechanism 60, and the base 58 is caused to reciprocate in the optical axis direction by the mirror moving mechanism 60. The optical path length of the reference light L2 is adjusted by the base 58 reciprocating in the optical axis direction. The mirror moving mechanism 60 includes, for example, a motor and a mechanism which converts output of the motor into reciprocating motion of the base 58. By the motor being driven, the base 58 periodically reciprocates relative to the first optical lens 52, whereby the optical path length of the reference light L2 periodically changes.

The optical probe 50 is operated by the examiner, and the distal end of the optical probe 50 can be inserted into an eyeball. The optical probe 50 includes an optical fiber coupler 26, and an optical probe body 40 connected to the optical fiber coupler 26. The optical fiber coupler 26 outputs the measurement light L1 from the optical fiber FB3, to the optical probe body 40. The optical probe body 40 guides the measurement light L1 from the optical fiber coupler 26, to a measurement target site, and guides the reflected light L1' from the measurement target site, to the optical fiber FB3. A second light source 92 is connected to the optical probe body 40, via an optical fiber FB5. The optical probe body 40 will be described in detail later.

The second light source 92 is a laser beam source (red laser beam source, for example) which emits visible light. Aiming light L3 outputted from the second light source 92 is inputted to the optical fiber coupler 26 via the optical fiber FB5. The optical fiber coupler 26 multiplexes the measurement light L1 from the optical fiber FB3 and the aiming light L3 from the optical fiber FB5, and outputs the multiplexed light (L1 + L3) to the optical probe body 40. Thus, the measurement light L1 and the aiming light L3 are applied to the same position as the measurement target site. In the present embodiment, the irradiation optical system 100 is comprised by the second light source 92 and the optical probe 50.

The interference optical system 10 described above is connected to the interference light detector 20 via the optical fibers FB6 and FB7. The interference light detector 20 is comprised by a light receiving element, for example, and converts interference light L4 inputted from the optical fibers FB6 and FB7, into an electric signal. The interference light detector 20 is connected to a tomographic image processing unit 22. The electric signal converted by the interference light detector 20 is inputted to the tomographic image processing unit 22.

The tomographic image processing unit 22 comprises: a signal processing circuit which processes the inputted signal; and an image processing unit which creates an image based on the processed signal. The tomographic image processing unit 22 uses an analysis result obtained based on the electric signal inputted from the interference light detector 20, to create an optical tomographic image of the measurement target eye 200 by a known method. The created optical tomographic image is outputted to a display unit 24 (monitor, for example), to be displayed on the display unit 24.

Next, the optical probe body 40 will be described in detail. The optical probe body 40 includes: an attachment unit 28 to which the optical fiber coupler 26 is attached; a grip unit 30 to be gripped by the examiner; a rotary unit 32 mounted to the grip unit 30; and a probe housing 34 mounted to the rotary unit 32. In the optical probe body 40 (that is, the attachment unit 28, the grip unit 30, the rotary unit 32, and the probe housing 34), an optical fiber being a transmission line through which light from the optical fiber coupler 26 is transmitted is disposed. This optical fiber is divided between the rotary unit 32 and the probe housing 34 such that the optical fiber on a probe housing 34 side is connected, via an optical rotary joint not shown, so as to be rotatable relative to the optical fiber on a rotary unit 32 side. The optical fiber on the probe housing 34 side is rotatable around the axis of the optical probe body 40 by the rotary unit 32.

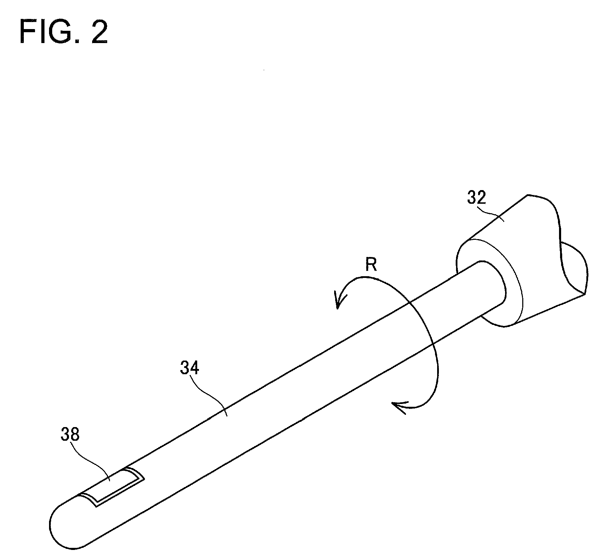

To the input end of the attachment unit 28, light from the optical fiber coupler 26 is inputted. The grip unit 30 is mounted to the output end of the attachment unit 28. The grip unit 30 is the portion to be gripped by the examiner when the examiner examines the measurement target eye 200. The rotary unit 32 comprises a mechanism (drive motor or the like) that causes the optical fiber on the probe housing 34 side to rotate. As shown in FIG 2, the probe housing 34 is a cylindrical member whose distal end is closed, and protrudes from the rotary unit 32. In order to make a hole small for inserting the probe housing 34 into an eyeball, the diameter of the distal end of the probe housing 34 is preferably small. On the other hand, from the viewpoint of operability, the probe housing 34 requires some rigidity. Thus, for example, the diameter can be set as φ 0.7 mm (23 gauge), φ 0.5 mm (25 gauge), or φ 0.3 mm (30 gauge). On the side face of the probe housing 34, an open window 38 is formed. The open window 38 is arranged in the vicinity of the distal end of the probe housing 34. The open window 38 is formed in a substantially rectangular shape, and is open in a predetermined angle range in the circumferential direction. The open window 38 is closed with a transmissive member, and water or the like from outside is prevented from entering therethrough.

In the probe housing 34, a reflective mirror 36 is disposed at a position corresponding to the open window 38. Specifically, the reflective mirror 36 is disposed at a position where the measurement light L1 and the aiming light L3 reflected by the reflective mirror 36 can pass through the open window 38. The reflective mirror 36 is mounted to the distal end of the optical fiber on the probe housing 34 side. Thus, the reflective mirror 36 is rotatable around the axis of the optical probe body 40 (that is, the optical axis of the measurement light L1 and the aiming light L3). The reflection surface of the reflective mirror 36 faces the rotary unit 32, and is inclined by 45 degrees relative to the axis of the optical probe body 40. Thus, the light L1 + L3 from the optical fiber coupler 26 is reflected at the reflection surface of the reflective mirror 36, to be applied to the outside of the probe housing 34 through the open window 38. Since the reflection surface of the reflective mirror 36 is inclined by 45 degrees relative to the axis of the optical probe body 40, the light reflected by the reflective mirror 36 is applied in a direction orthogonal to the axis of the optical probe body 40. In addition, since the reflective mirror 36 rotates, the application direction of the light reflected by the reflective mirror 36 changes in the circumferential direction in accordance with lapse of time. Since the open window 38 is open in a predetermined angle range in the circumferential direction, the range in which the light reflected by the reflective mirror 36 is applied is also limited to the predetermined angle range in the circumferential direction. In the present embodiment described above, the rotary unit 32 is arranged in the optical probe body 40, but without being limited thereto, the rotary unit may be arranged outside the body 40. More specifically, the rotary unit 32 is arranged between the optical fiber coupler 26 and the body 40, and the optical fiber on the probe housing 34 side relative to the rotary unit 32 and the reflective mirror 36 mounted to the distal end of this optical fiber are caused to rotate by being driven by the rotary unit 32. The optical fiber coupler 26 and the rotary unit 32 are provided as separate members without being integrated with the body 40, and the rotary unit 32 and the body 40 are connected to each other by means of an optical fiber having a sufficient length that can cause a user to grip only the body 40 in handling. Preferably, the optical fiber between the rotary unit 32 and the body 40 is surrounded by a flexible hollow tube to be protected from the outside. It should be noted that the hollow tube is connected between the rotary unit 32 and the body 40 so as not to be rotated by the rotary unit 32, and the optical fiber passing through the hollow tube is rotated by the rotary unit 32. In this configuration, since the rotation mechanism is not provided to the body 40, the body 40, which is the portion to be gripped by the user, can be downsized.

In the present embodiment, the light L1 + L3 is reflected toward the outside of the probe housing by use of the reflective mirror, but without being limited thereto, a prism can be used as a reflective member instead of the reflective mirror. Moreover, the inclination angle of the reflective mirror (reflective member) is not limited to 45 degrees, but may be any inclination angle that can cause a desired light emission direction to be obtained.

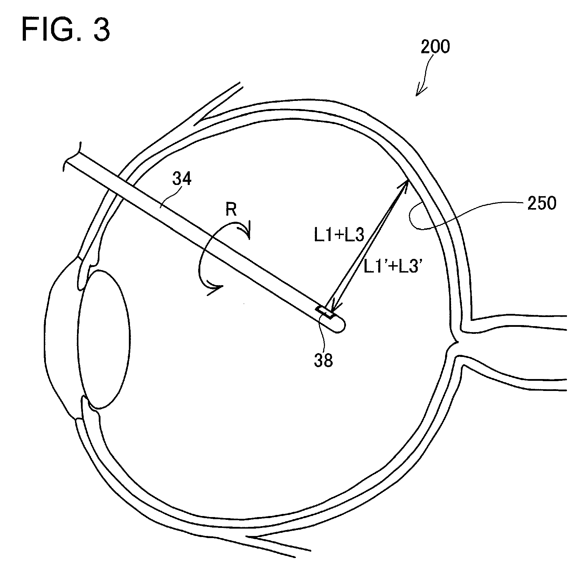

Next, the procedure of examining the measurement target eye 200 by using the optical tomography device of the present embodiment will be described. The optical tomography device of the present embodiment is used when surgery is performed on an eye of a patient. For example, as shown in FIG. 3, an incised wound for surgery is formed in the measurement target eye 200, and the probe housing 34 of the optical probe 50 is inserted through the incised wound into the eyeball. Next, the first light source 12 and the second light source 92 are turned on, and drive of the mirror moving mechanism 60 and the rotary unit 32 are started. Accordingly, interference light is detected by the interference light detector 20, an optical tomographic image of the measurement target eye 200 is created based on the detected interference light, and the created optical tomographic image is displayed on the display unit 24.

That is, the light L outputted from the first light source 12 passes through the optical fiber FB1, the optical fiber coupler 14, and the optical fiber FB2, so as to be inputted to the optical fiber coupler 16. The light L inputted to the optical fiber coupler 16 is split into the reference light L2 and the measurement light L1.

The reference light L2 passes through the optical fiber FB4 so as to be inputted to the reference optical system 90. The reference light L2 inputted to the reference optical system 90 passes through the first optical lens 52 and the second optical lens 54, so as to be applied to the reflective mirror 56. Reference light L2' reflected by the reflective mirror 56 passes through the second optical lens 54 and the first optical lens 52 so as to be guided to the optical fiber FB4. The reference light L2' guided to the optical fiber FB4 is inputted to the optical fiber coupler 16. Here, the second optical lens 54 and the reflective mirror 56 periodically reciprocate relative to the first optical lens 52 by the mirror moving mechanism 60. Thus, the optical path length of the reference light L2 also periodically changes.

On the other hand, the measurement light L1 passes through the optical fiber FB3, so as to be inputted to the optical fiber coupler 26 of the optical probe 50. To the optical fiber coupler 26, the aiming light L3 from the second light source 92 is also inputted. Thus, the measurement light L1 and the aiming light L3 are combined by the optical fiber coupler 26. The combined light L1 + L3 is inputted to the optical fiber in the optical probe body 40. The light L1 + L3 inputted into the optical probe body 40 is reflected by the reflective mirror 36, so as to be applied to a measurement target site (retina 250, for example) of the measurement target eye 200 through the open window 38 in the probe housing 34. Here, since the reflective mirror 36 is rotated by the rotary unit 32, the light L1 + L3 which is applied to the measurement target site is rotated around the axis of the optical probe body 40. On the other hand, the open window 38 formed in the probe housing 34 is open only within the predetermined angle range in the circumferential direction of the probe housing 34, and thus, the range in which the light L1 + L3 to be applied to the measurement target site is limited to the predetermined angle range. Since the rotation of the reflective mirror 36 is periodically performed, the position, in the measurement target site, to which the light is applied also periodically changes. That is, the light L1 + L3 applied to the measurement target site scans in the predetermined range.

The light (L1 + L3) applied to the measurement target site of the measurement target eye 200 is reflected at the measurement target site. The reflected light (L1' + L3') passes through the open window 38 to be guided into the probe housing 34. The light (L1' + L3') guided into the probe housing 34 passes through the optical fiber coupler 26 and the optical fiber FB3, so as to be inputted to the optical fiber coupler 16. To the optical fiber coupler 16, the light (L1' + L3') from the optical probe 50 and the reference light L2' from the reference optical system 90 are inputted. The optical fiber coupler 16 combines the inputted light (L1' + L3') and light L2' into an interference wave L4. The interference wave L4 passes through the optical fibers FB2, FB6, and FB7, so as to be detected by the interference light detector 20, and the tomographic image processing unit 22 creates an optical tomographic image of the measurement target eye 200 based on the detected interference light. The created optical tomographic image is displayed on the display unit 24. Accordingly, the examiner can determine the state of the measurement target site (retina 250) of the measurement target eye 200, based on the optical tomographic image displayed on the display unit 24. Accordingly, the examiner can provide necessary treatment.

In the optical tomography device of the present embodiment, the aiming light is applied to the position where the measurement light is applied. Thus, the examiner can confirm, with one's naked eyes, the position to which the aiming light is being applied, and can obtain an optical tomographic image of the desired position while confirming the aiming light. Accordingly, the distal end of the optical probe 50 can be prevented from coming into contact with internal tissue of the measurement target eye 200. In addition, the range in which the open window 38 is open in the probe housing 34 is limited to the predetermined angle range in the circumferential direction, whereby the site to which the measurement light and the aiming light are applied is limited. This also can cause the examiner to obtain an optical tomographic image of the site as intended, and thus, to accurately diagnose the state of the measurement target eye 200.

### (Specific example)

Next, optical tomographic images captured by using the optical tomography device of the present embodiment will be explained. This image capturing was performed after obtaining approval by the ethics committee of Nagoya University.

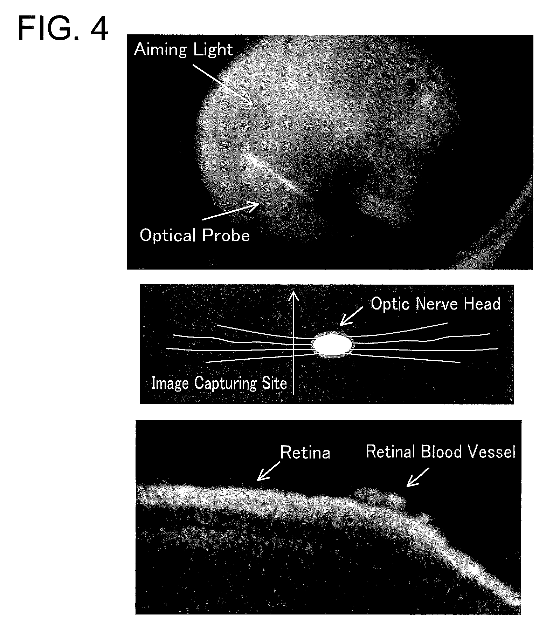

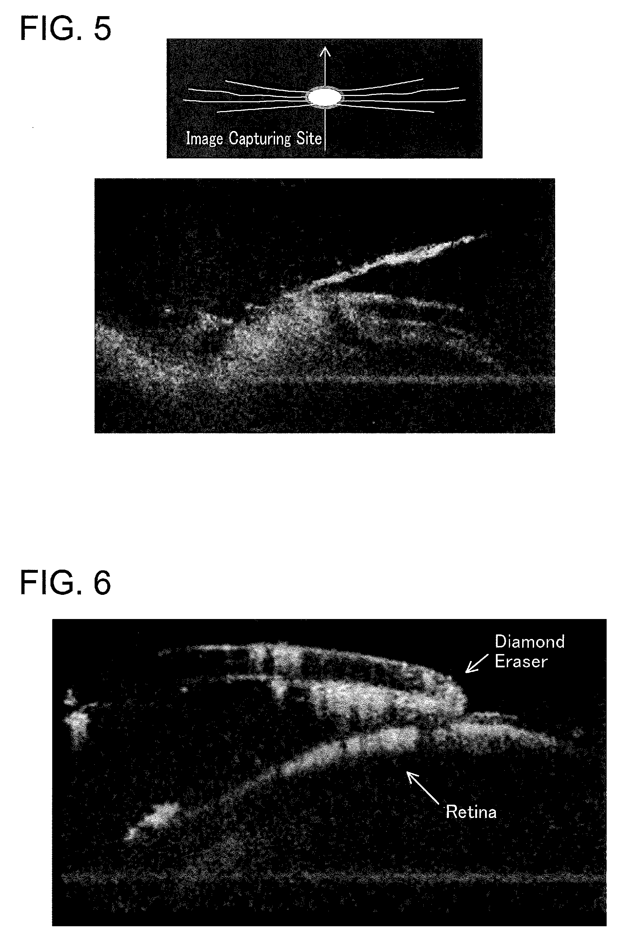

FIG. 4 shows an optical tomographic image of a normal retina of a domestic rabbit eye. More specifically, the top image in FIG 4 is an image of the inside of the eyeball at the time of capturing the optical tomographic image. This image shows the state where the aiming light is being applied from the optical probe of the optical tomography device. The middle image in FIG. 4 schematically shows the image capturing site of the optical tomographic image. The bottom image in FIG 4 is an optical tomographic image at the site where the aiming light is applied. In the optical tomographic image, the upper side shows the surface layer, and the lower side shows the deeper layer. In the optical tomographic image, the retina and the retinal blood vessels that extend on the retina surface layer are captured. FIG 5 shows an optical tomographic image of an optic nerve head of the domestic rabbit eye. In this manner, with the optical tomography device of the present embodiment, it is possible to appropriately capture an optical tomographic image of the targeted tissue in the eyeball.

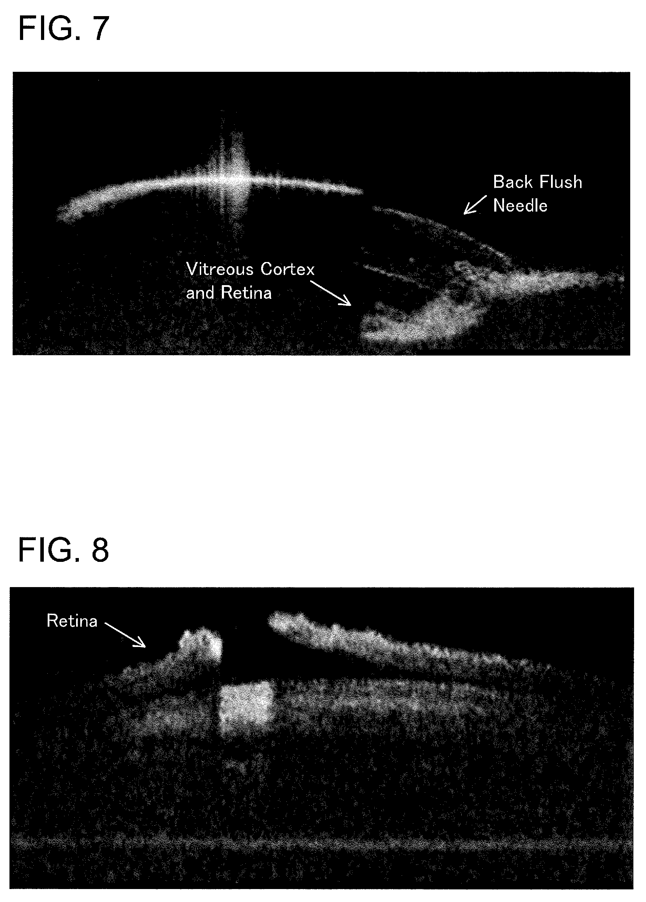

FIG. 6 and FIG. 7 each show an optical tomographic image of a normal retina of a domestic rabbit eye. In the optical tomographic image shown in FIG 6, a diamond eraser is in contact with the surface layer of the normal retina. In the optical tomographic image shown in FIG. 7, the vitreous cortex and a part of the retina are aspirated and pulled by a back flush needle. It should be noted that the images in FIG 6 and FIG. 7 are static images here, but actually, have been taken as a video image. Thus, the examiner can operate a surgery instrument such as a diamond eraser or a back flush needle etc., while viewing the optical tomographic video image of the tissue.

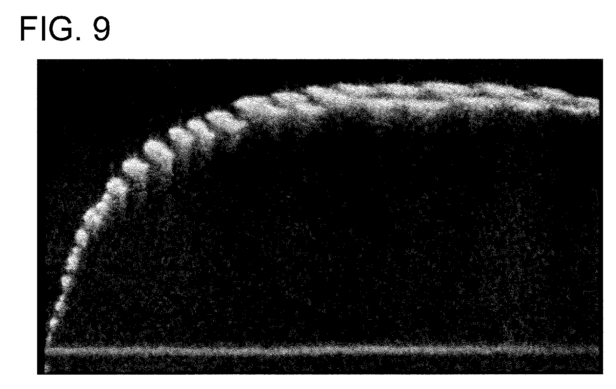

FIG. 8 shows an optical tomographic image of the retina of a domestic rabbit eye. This optical tomographic image reveals the state where the retina has been detached. FIG. 9 shows an optical tomographic image of the pars plicate ciliaris of a pig eye. The pars plicate ciliaris is behind the iris when viewed from outside the eyeball. Thus, it is difficult to capture an optical tomographic image of the pars plicate ciliaris, from outside the eyeball. In the present embodiment, since an optical tomographic image is captured from inside the eyeball, it is possible to obtain an optical tomographic image of the pars plicate ciliaris as shown in FIG. 9.

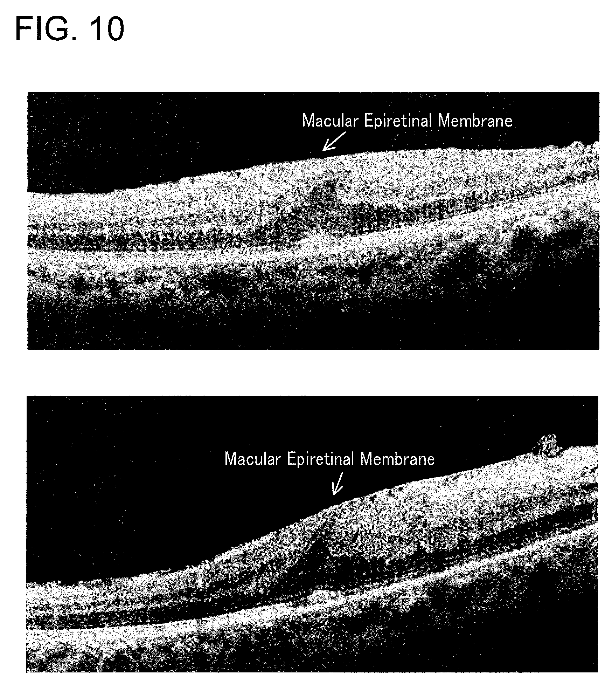

FIG. 10 shows an optical tomographic image of a human epiretinal membrane with macular epiretinal membrane disease. The optical tomographic image shown in FIG. 10 was captured during ambulatory examination. The image capturing site (scan line along which the aiming light scans) in the upper optical tomographic image in FIG. 10 is orthogonal to the image capturing site (scan line along which the aiming light scans) in the lower optical tomographic image in FIG 10. With reference to the optical tomographic images, there is a membrane on the retina in the macular area which is the center portion of the retina, and the retina has swelled.

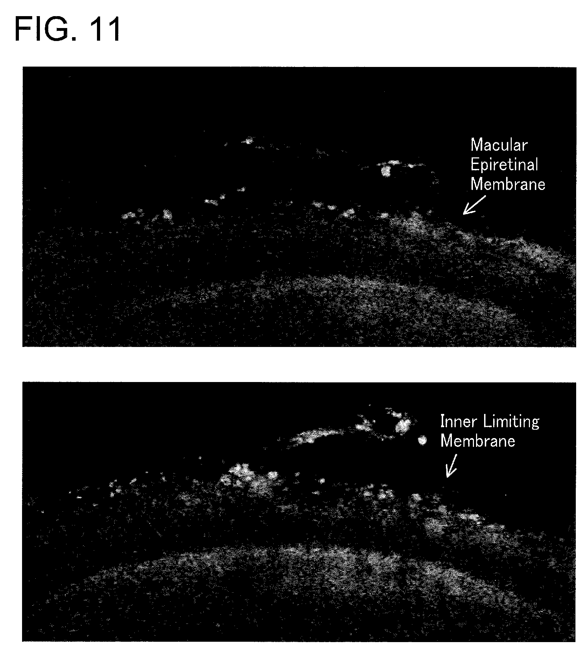

FIG. 11 shows optical tomographic images of the epiretinal membrane shown in FIG. 10 observed during surgery. Specifically, the upper image and the lower image in FIG. 11 respectively show the macular epiretinal membrane and the inner limiting membrane being detached with tweezers. In this manner, with the optical tomography device of the present embodiment, it is possible to appropriately capture images in the surgery being performed.

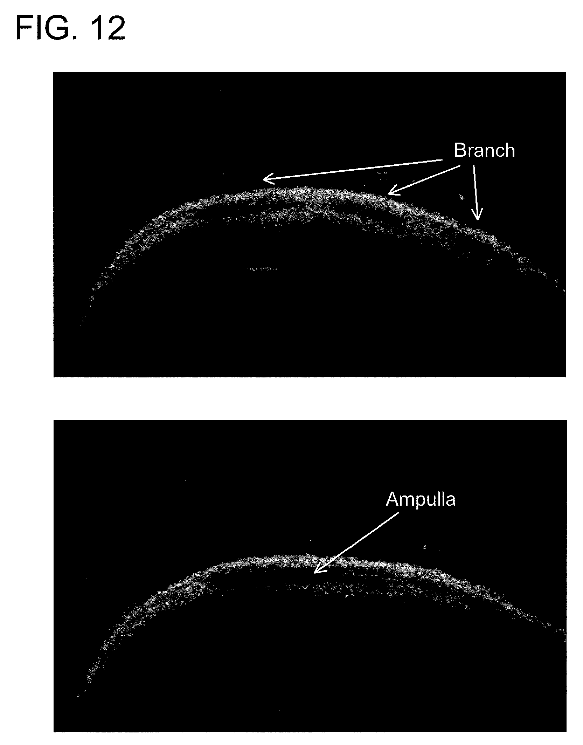

FIG. 12 shows optical tomographic images of vortex veins in the choroid under human retina. More specifically, the upper image and the lower image in FIG. 12 respectively show optical tomographic images of vortex vein branches and a vortex vein ampulla.

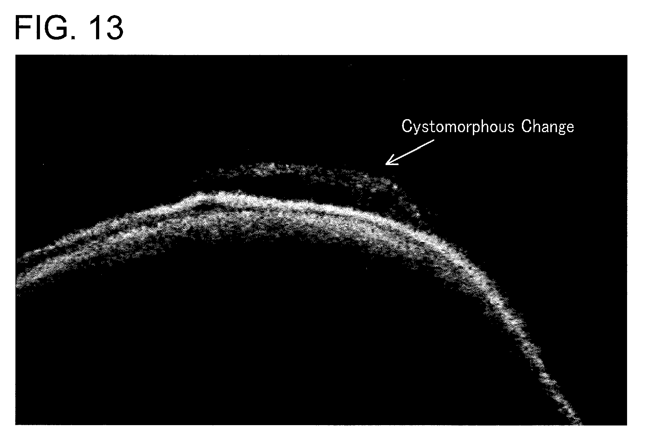

FIG. 13 shows an optical tomographic image of the ora serrata retinae which is the most peripheral part of human retina. In the ora serrata retinae, cystomorphous change is observed.

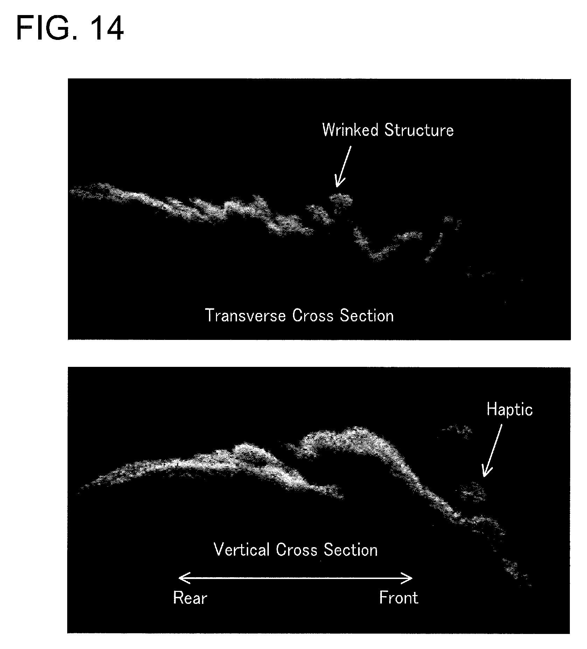

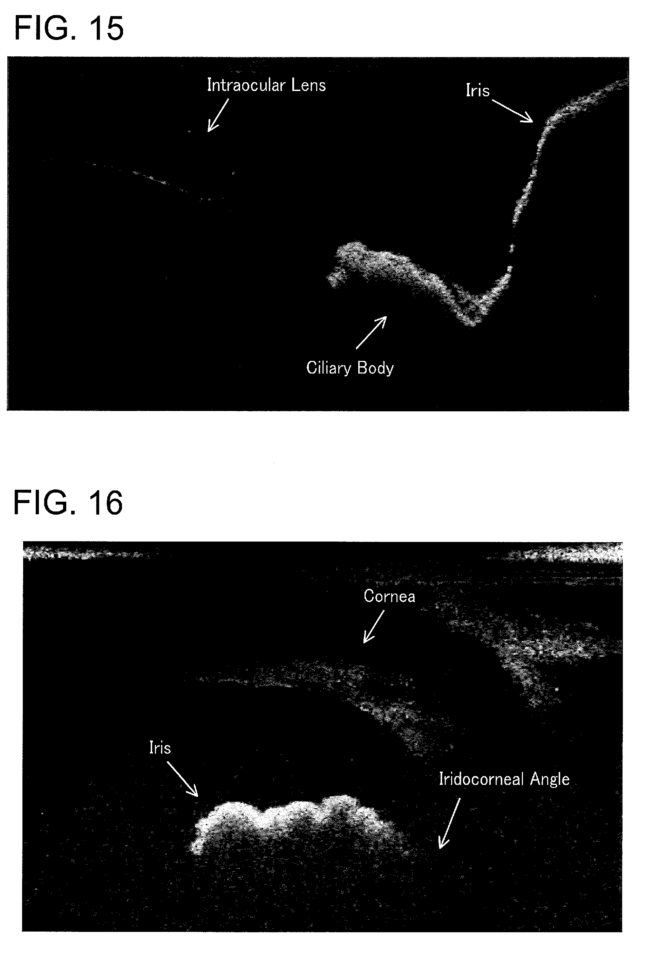

FIG. 14 shows optical tomographic images of human ciliary body. More specifically, the upper image and the lower image in FIG 14 respectively show optical tomographic images of a transverse cross section and a vertical cross section of the ciliary body. In the optical tomographic image in the transverse cross section, a wrinkled structure of the ciliary body is visualized, and in the optical tomographic image in the vertical cross section, the haptic is visualized. FIG 15 shows an optical tomographic image of human intraocular lens, ciliary body, and iris. The images shown in FIG. 14 were captured by inserting the optical probe through a side port in conjunctiva, whereas the image shown in FIG. 15 was captured by inserting the optical probe through a side port in the anterior segment of the eyeball. That is, in FIG. 15, the image was captured at a position nearer to the front of the eye, than in FIG. 14. Thus, the image in FIG. 15 includes the intraocular lens and the iris, in addition to the ciliary body.

FIG. 16 shows an optical tomographic image of human cornea, iris, and iridocorneal angle. The image shown in FIG. 16 has been captured from outside the eyeball, without inserting the optical probe into the eyeball.

Specific examples of the present invention have been described in detail, however, these are mere exemplary indications and thus do not limit the scope of the claims. The art described in the claims includes modifications and variations of the specific examples presented above. Modifications of the above embodiment are listed below.

The optical tomography device of the present embodiment is an OCT device of a time-domain type which drives the reflective mirror 56, but may be an SD-OCT device which uses a broadband light source, or may be an SS-OCT device which uses a wavelength-swept light source. The SD-OCT device or the SS-OCT device does not require driving of the reflective mirror 56 during measurement, and thus, can obtain optical tomographic images in a short time.

In the present embodiment, reflected light L3' of the aiming light L3 passes through the opening 38 to be guided to the interference light detector 20. However, instead of this, the reflected light L3' may be cut by an optical filter. In this case, the reflected light L3' is not guided to the interference light detector 20.

In the present embodiment, the measurement light from the light source 12 is sent to the optical fiber coupler 16 by using the optical fiber coupler 14, and the interference wave L4 obtained through combining in the optical fiber coupler 16 is guided to the interference light detector 20 via the optical fiber coupler 14, however, without being limited thereto. A circulator may be used instead of the optical fiber coupler 14, so as to efficiently guide the interference wave L4 to the interference light detector 20. More specifically, by using a circulator instead of the optical fiber coupler 14 shown FIG. 1, the measurement light from the light source 12 is sent to the optical fiber coupler 16 via the circulator, and the interference wave L4 from the optical fiber coupler 16 is guided to the interference light detector 20. In such a configuration, none of the interference wave L4 returns to the light source 12 side, and thus, the interference wave can be guided to the interference light detector 20 with optical loss reduced.

Alternatively, the following configuration is also conceivable: the measurement light from the light source 12 is directly sent to the optical fiber coupler 16 without using the optical fiber coupler 14; circulators are respectively disposed between the optical fiber coupler 16 and the reference optical system 90 and between the optical fiber coupler 16 and the optical probe 50; reflected lights (L2', and L1' + L3') entering the respective circulators are combined by a new optical fiber coupler to obtain an interference wave; and then, the interference wave is distributed to be guided to the interference light detector 20. Also in this configuration, the interference wave can be guided to the interference light detector 20 with optical loss reduced.

The second light source for emitting the aiming light may be configured capable of emitting light of a plurality of colors. For example, the second light source 92 may comprise: a red light source unit which emits red light (wavelength: 600 to 780 nm); and a green light source unit which emits green light (wavelength: 500 to 570 nm). In such a case, the optical tomography device can comprise a selecting switch which selects either light from the red light source unit or light from the green light source unit as the light to be applied to an eye to be examined. For example, the optical tomography device is configured such that either of a filter that removes green light or a filter that removes red light can be disposed on the optical path thereof. When the examiner operates the selecting switch, either one of the filters is disposed on the optical path, whereby either green light or red light is applied to the eye to be examined. In such a configuration, green aiming light or red aiming light can be selectively applied to the eye to be examined. Since the color of the tissue in the eyeball is close to red (i.e., complementary color of green), if green light is selected as the aiming light, the examiner can easily recognize the applied aiming light L3. On the other hand, if red light is selected as the aiming light, compared with the case where green light is selected, influence on the tissue in the eyeball caused by application of the aiming light L3 can be reduced. Thus, the color of the aiming light to be applied to the eye to be examined may be selected as appropriate in accordance with the purpose.

The technical elements described in the present specification or in the drawings exhibit technical utility singly or in various combinations and are not limited to the combinations recited in the claims as filed. Moreover, the techniques illustrated in the present specification or in the drawings simultaneously attain a plurality of purposes, and attaining one of the purposes per se offers technical utility.

### Reference Signs List

10 Interference Optical System
12 Light Source
14 Optical Fiber Coupler
16 Optical Fiber Coupler
20 Interference Light Detector
22 Tomographic Image Processing Unit
24 Display Unit
26 Optical Fiber Coupler
28 Attachment Unit
30 Grip Unit
32 Rotary Unit
34 Probe Housing
36 Reflective Mirror
38 Open Window
40 Optical Probe Body
50 Optical Probe
52 First Optical Lens
54 Second Optical Lens
56 Reflective Mirror
58 Base
60 Mirror Moving Mechanism
90 Reference Optical System
92 Light Source
100 Irradiation Optical System
200 Measurement Target Eye
250 Retina
Fb1 To Fb7 Optical Fiber

## Claims

1. An optical tomography device comprising:
a first light source; and
an optical device configured to guide first light from the first light source to a measurement target eye and detect interference light obtained from reflected light from the measurement target eye,
the optical tomography device further comprising:
a second light source configured to emit aiming light indicative of a position to which the first light is applied; and
an optical probe including an opening at a distal end and configured capable of being inserted in an eyeball, wherein
the optical probe applies the first light guided by the optical device, into the measurement target eye through the opening, guides the reflected light, which has been reflected by the measurement target eye, to the optical device, and applies, though the opening, the aiming light from the second light source to the position to which the first light is applied.

2. The optical tomography device according to claim 1, wherein
the opening in the optical probe is arranged on a side face of the optical probe in a predetermined angle range in a circumferential direction, and
the first light and the aiming light are applied, through the opening, in a direction orthogonal to an axis of the optical probe, and scan in the predetermined angle range around the axis of the optical probe.

3. The optical tomography device according to claim 1 or 2, wherein
the second light source is configured capable of emitting the aiming light of a plurality of colors, and
the optical tomography device further comprises a selecting switch configured to select a luminescent color of the aiming light to be emitted from the second light source to the measurement target eye.

4. The optical tomography device according to claim 3, wherein
the second light source is configured capable of emitting red-colored aiming light and green-colored aiming light.
